Europäisches Patentamt

European Patent Office

Office européen des brevets

(19)

(11) **EP 0 664 684 B1**

(12) **EUROPÄISCHE PATENTSCHRIFT**

(45) Veröffentlichungstag und Bekanntmachung des Hinweises auf die Patenterteilung:
**28.01.1998 Patentblatt 1998/05**

(21) Anmeldenummer: **93922499.4**

(22) Anmeldetag: **14.10.1993**

(51) Int. Cl.$^6$: **A61B 8/10**

(86) Internationale Anmeldenummer:
**PCT/DE93/00992**

(87) Internationale Veröffentlichungsnummer:
**WO 94/08510 (28.04.1994  Gazette 1994/10)**

(54) **VORRICHTUNG ZUR UNTERSUCHUNG DES AUGES, INSBESONDERE DES MENSCHLICHEN AUGES**

DEVICE FOR EXAMINING THE EYE, IN PARTICULAR THE HUMAN EYE

DISPOSITIF PERMETTANT D'EXAMINER L'OEIL, NOTAMMENT L'OEIL HUMAIN

(84) Benannte Vertragsstaaten:
**AT BE CH DE DK ES FR GB GR IE IT LI LU NL PT SE**

(30) Priorität: **17.10.1992 DE 4235079**

(43) Veröffentlichungstag der Anmeldung:
**02.08.1995  Patentblatt 1995/31**

(73) Patentinhaber: **TOMEY AG**
**D-91058 Erlangen-Tennenlohe (DE)**

(72) Erfinder: **OBERMAIER, Marco**
**D-81667 München (DE)**

(74) Vertreter:
**Voigt, Günter, Dipl.-Ing.**
**Patentanwälte Dr. Schulze & Voigt**
**Nordring 152**
**90119 Nürnberg (DE)**

(56) Entgegenhaltungen:
WO-A-90/12534          AT-A- 227 868
US-A- 3 597 964        US-A- 3 948 248
US-A- 4 930 507

• ACOUSTICAL IMAGING, Bd.14, 1985, NEW YORK (US) Seiten 1 - 17 F.L. LIZZI ET AL. 'Ultrasonic Therapy and Imaging in Ophthalmology'

## Beschreibung

Die Erfindung bezieht sich auf eine Vorrichtung mit Ultraschall- oder Laser-Meßsonde zur Untersuchung des Auges und zum Messen und Erfassen von Funktionen und Zuständen desselben unter Verwendung eines auf das Auge aufsetzbaren Halters von im wesentlichen kegelstumpfförmiger Gestalt, wobei sich der Kegel in Richtung auf das Auge konisch verjüngt, und augenseitig eine der Krümmung des Augapfels entsprechende konkave Ausnehmung als Auflagefläche aufweist.

Die bekannten Methoden zur Untersuchung des Auges, insbesondere zur präoperativen Untersuchung, sind für den Arzt und Diagnostiker verhältnismäßig unsicher und recht ungenau. Sie liefern insbesondere auch keine wiederholt meßbare und untereinander vergleichbare Werte.

Die Beurteilung und Quantifizierung der retinalen Durchblutung mit der sogenannten indirekten Ophthalmoskopie liefert keine zuverlässigen und reproduzierbaren Meßwerte.

Aus der AT-A-227 868 ist eine Vorrichtung zur Untersuchung des menschlichen Auges und zum Erfassen von Funktionen und Zuständen desselben mit einer Meßsonde bekannt, die in einem auf das Auge aufsetzbaren Halter angeordnet ist. Dabei wird ein Ultraschallwandler verwendet. Die augenseitige Linsenfläche der bekannten Vorrichtung ist entsprechend der Krümmung des Augapfels konkav gewölbt. Diese bekannte Vorrichtung arbeitet als Ultraschall-Echolot.

Aus der US-A-4 823 801 sind zwei Ultraschall-Meßsonden bekannt, deren Meßrichtung (Strahlungsrichtung) entlang der Mantelfläche des Kegels verläuft. Diese Meßsonden sind Teile einer Matrix von vielen Sonden. Sie arbeiten nicht nach dem Doppler-Prinzip, sondern nach dem Echo-Laufzeit-Prinzip und messen nicht die Blutgeschwindigkeit, sondern die Hornhautdicke unter der gesamten Matrix.

Aus der US-A-5 032 020 ist ferner eine Vorrichtung zum Untersuchen des menschlichen Auges bekannt, bei der in einem als Kontaktglas ausgebildeten Halter ein Drucksensor angeordnet ist, der die Messung des Augeninnendrucks ermöglicht.

Aus der US-A-5 109 852 ist eine Vorrichtung mit einem speziellen Drucksensor bekannt, der durchsichtig ist und vom Patienten über einen längeren Zeitraum getragen werden kann, so daß eine kontinuierliche Messung des Augeninnendrucks möglich ist.

Aus der US-A-4 823 801 ist eine Vorrichtung zum Untersuchen von Zuständen des menschlichen Auges bekannt, mit der die Dicke der Hornhaut gemessen wird.

Aus der US-A-4 485 820 ist weiter eine Vorrichtung zum kontinuierlichen Erfassen der Hämoglobin-Sättigung im Blut von Frühgeborenen bekannt, wobei diese Vorrichtung einen auf das Auge aufsetzbaren Halter aufweist, in dem als Sonden Lichtleiter enden, die zu einer Lichtquelle oder zu Fotodedektoren in einem Auswertegerät führen.

Schließlich ist noch aus der DE-A-2 639 635 eine Vorrichtung zum Ableiten des Elektro-Retinogramms mit einem auf das Auge aufsetzbaren Halter bekannt, in dem als Sonden elektrische Kontakte zum Abnehmen der Potentiale angebracht sind.

Der Erfindung liegt die Aufgabe zugrunde, eine Vorrichtung zu schaffen, mit der alle wesentlichen Durchblutungsparameter des Auges zuverlässig und reproduzierbar gemessen und erfaßt werden können.

Diese Aufgabe wird erfindungsgemäß dadurch gelöst, daß bei einer Vorrichtung der eingangs erwähnten Art innerhalb des Halters und im wesentlichen parallel zur Mantelfläche des Kegelstumpfes eine Meßsonde angeordnet ist, die als Sender unnd Empfänger für impulsförmige Signale ausgebildet ist und neben einem Impulsgeber auch eine zur Erfassung des Doppler-Effekts geeignete Auswerteeinrichtung aufweist.

Vorteilhafte Weiterbildungen ergeben sich aus den Unteransprüchen.

Mit der erfindungsgemäßen Vorrichtung ist es möglich, die Durchblutung des menschlichen Auges und auch die Durchströmrichtung des Blutes durch das menschliche Auge genau zu messen. Ferner ist es möglich, gleichzeitig zusätzlich den Blutdruck im Auge zu messen. Beide Messungen waren bisher nicht oder nur ungenau durchzuführen, da die hierfür verwendeten Sonden nicht genau auf das Auge fixiert werden konnten.

Ein weiterer Vorteil der erfindungsgemäßen Vorrichtung liegt darin, eine funktionale Blutflußdiagnose in unklaren bzw. verschleierten Medien durchzuführen, beispielsweise für die präoperative Diagnose und für die Prognose der Kataraktchirurgie.

Schließlich - aber keinesfalls abschließend - ist es möglich, gleichzeitig eine Kontaktspiegelfundoskopie durchzuführen.

All diese Vorteile und Möglichkeiten werden insbesondere dadurch erreicht, daß die erfindungsgemäße Vorrichtung eine Meßsonde aufweist, die in oder an einem auf das Auge aufsetzbaren Halter angeordnet ist. Die Meßsonde ist eine Ultraschall-Doppler-Meßsonde oder eine Laser-Doppler-Meßsonde, während der Halter ein Körper aus Glas oder sonstigem (nicht unbedingt durchsichtigen) Material wie Kunststoff ist, der einen Fuß aufweist, welcher einer der äußeren Wölbungen des Auges und insbesondere des menschlichen Auges angepaßte Auflagefläche aufweist.

Für die Messung des inneren Druckes eines Auges, der nicht zuletzt auch vom Blutdruck abhängt, kann zusätzlich ein Drucksensor in den Halter eingebaut sein, der mehr oder weniger direkt mit der für die Messung zur Verfügung stehenden Oberfläche des menschlichen Auges in Berührung gebracht werden kann.

Dadurch ergibt sich die Möglichkeit, die Meßsonde und gleichzeitig auch den Drucksensor genau zu plazieren, um so exakte und auch wiederholbare Messungen durchführen zu können.

In der Zeichnung ist ein Ausführungsbeispiel einer erfindungsgemäßen Vorrichtung zum Untersuchen menschlicher Augen schematisch dargestellt, und zwar zeigt:

Fig. 1      eine schaubildliche Ansicht der auf ein menschliches Auge aufgesetzten erfindungsgemäßen Vorrichtung in einem senkrechten Schnitt und

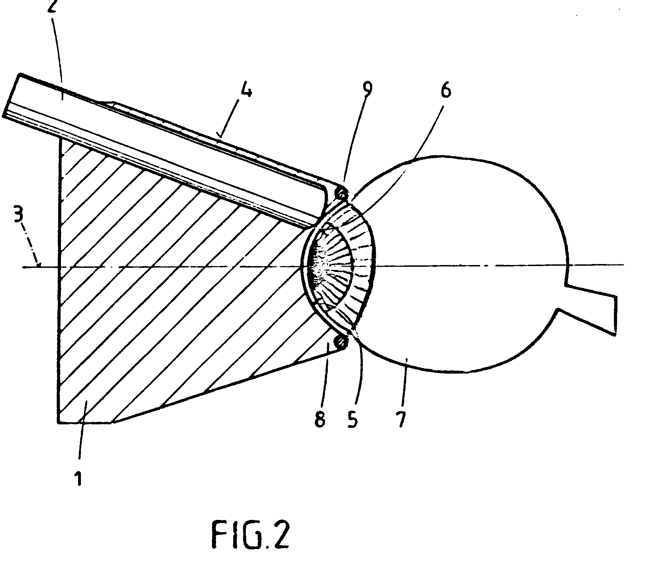

Fig. 2      eine Ansicht, ähnlich wie in Fig. 1 geschnitten, der erfindungsgemäßen Vorrichtung, die auf ein menschliches Auge aufgesetzt ist.

Die in der Zeichnung dargestellte Vorrichtung besteht aus einem im wesentlichen kegelstumpfförmigen Halter 1, in den eine für Meßzwecke bestimmte Sonde 2 eingelassen ist, und zwar derart, daß sie zur zentralen Achse 3 des kegelstumpfförmigen Halters 1 geneigt verläuft, vorzugsweise parallel zur kegeligen Mantelfläche 4 des Halters 1. Die Sonde ist auf die Eintrittsstelle der Augenarterie in den Augapfel ausgerichtet. Dies ist eine wichtige Voraussetzng für die durchzuführenden Messungen.

Die Sonde 2 kann eine Doppler-Sonde, d.h. eine Schall- bzw. Ultraschall-Sonde, sein, aber ebenso eine Laser-Sonde. Für die Erfindung ist es wichtig, daß die Sonde 2 mittels des auf ein Auge - wie insbesondere ein menschliches Auge - aufsetzbaren Halters 1 für Meß- und sonstige Untersuchungszwecke genau und wiederholbar positioniert werden kann. Zu diesem Zweck hat der Halter 1 in seinem Fußbereich eine konkav nach innen gehende kugelförmige Auflagefläche 5, welche der äußeren Wölbung 6 des zu untersuchenden Auges 7 angepaßt ist. Daher kann der Halter 1 mit der darin untergebrachten Sonde 2 unmittelbar auf das Auge 7 aufgesetzt werden, um die gewünschten Messungen durchzuführen.

Durch die besondere Anordnung der Doppler-Sonde 2 im Kontaktglas des Halters 1 wird das Meßvolumen am Sehnervkopf beim Aufsetzen auf das Auge schnell und sicher erfaßt, weil aufgrund der Anordnung der Sondenachse und die in diesem Zusammenhang relevanten Gefäße des Auges in einer Fluchtlinie liegen. Dies hat zur Folge, das der Cosinus in der nachfolgenden Formel den Wert "1,0" annimmt und somit die gemessene Geschwindigkeit der wirklichen Doppler-Verschiebung entspricht, wodurch erstmals vergleichbare Messungen möglich werden.

$$f_{Doppler} = k \cdot \cos a \cdot v_{Blut}$$

Dabei ist:

$f_{Doppler}$      = Dopplerfrequenz
$k$      = Konstante

$a$      = Winkel zwischen Blutflußrichtung und Sondenachse
$v_{Blut}$      = Blutflußgeschwindigkeit

Im äußeren und dem Auge 7 zugewandten umlaufenden Rand 8 des Halters 1 ist beim dargestellten Ausführungsbeispiel ein ringförmiger Drucksensor 9 mit insgesamt hinreichend großer Auflagefläche angeordnet, mit dessen Hilfe der innere Druck im Auge 7 gemessen werden kann, wenn der Halter 1 auf das Auge 7 aufgesetzt ist. Die hinreichend große Auflagefläche ist sehr wichtig, da zur später noch im einzelnen zu beschreibenden Blutdruckmessung der Anpreßdruck des Halters 1 und damit auch des ringförmigen Drucksensors 9 auf den Augapfel kurzzeitig erhöht werden muß. Bei einer zu kleinen Auflagefläche bestände die Gefahr einer Schädigung des Augapfels.

Zur Blutdruckmessung wird das Kontaktglas bzw. der Halter 1 derart auf das Auge aufgesetzt, daß die Sondenachse genau auf die Eintrittsstelle der Augen-Arterie 11 in den Augapfel ausgerichtet ist. Dies kann bei einem durchsichtigen Halter 1 vom Arzt optisch überprüft werden. Bei einem undurchsichtigen Halter 1 läßt sich die Ultraschall-Sonde nach der Qualität des Doppler-Signals optisch und akustisch feststellen. Die richtige Ausrichtung ist gegeben, wenn die größte Entfernung nach dem Echolot-Verfahren ermittelt wird.

Durch Aufpressen des Halters 1 auf den Augapfel wird sodann der Innendruck des Augapfels solange erhöht, bis mit der Ultraschall-Doppler-Sonde gerade kein Signal mehr detektiert wird. Dies bedeutet, daß der so applizierte Druck dem systolischen Blutdruck der Arteria centralis retinae entspricht. Wird die zuvor erfolgte Druckerhöhung wieder rückgängig gemacht, erreicht man schließlich den diastolischen Druck, bei dem das Blut in der Vena centralis retinae wieder zu pulsieren beginnt.

Im Zusammenwirken von Doppler-Sonde 2 und Drucksensor 9 kann somit auch eine sehr genaue und reproduzierbare Blutdruckmessung erfolgen.

Als Drucksensor kommen beispielsweise eine Piezofolie oder ein Piezokristall in Frage, die vorzugsweise ringförmig am Halter 1 angeordnet sind.

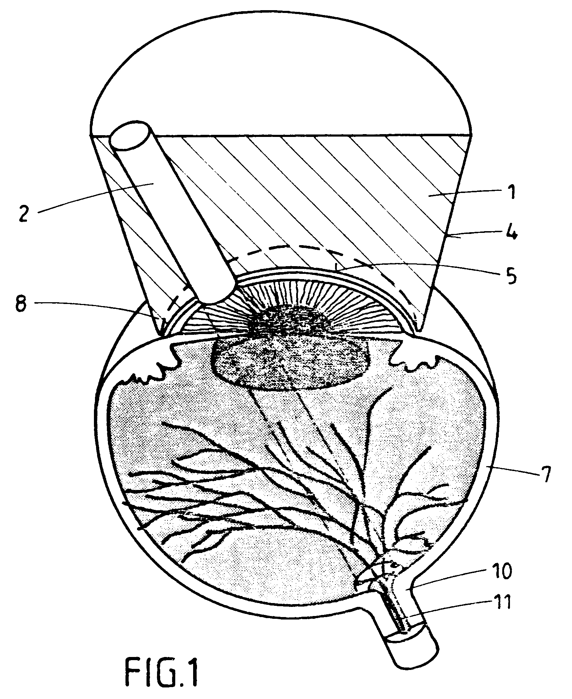

Mit der erfindungsgemäßen Vorrichtung kann man, wie insbesondere Fig. 1 zeigt, die zum Messen bestimmte Sonde 2 auf ein menschliches Auge 7 derart aufsetzen, daß diese Sonde 2 exakt auf den Eintritt 10 für den Sehnerv und die dort befindliche Augen-Arterie 11 des Auges ausgerichtet ist.

Erst damit sind dann vergleichbare und reproduzierbare Meßwerte erreichbar, wie sie insbesondere für Langzeit-Diagnosen unabdingbare Voraussetzung sind. Nur so kann der Arzt nämlich die sich über längere Zeiträume ergebenden Veränderungen im Auge anhand der erstellten Diagramme zuverlässug miteinander vergleichen und Aussagen über den Krankheitsverlauf treffen.

## Patentansprüche

1. Vorrichtung mit Ultraschall- oder Laser-Meßsonde zur Untersuchung des Auges und zum Messen und Erfassen von Funktionen und Zuständen desselben unter Verwendung eines auf das Auge aufsetzbaren Halters von im wesentlichen kegelstumpfförmiger Gestalt, wobei sich der Kegel in Richtung auf das Auge konisch verjüngt, und augseitig eine der Krümmung des Augapfels entsprechende konkave Ausnehmung als Auflagefläche aufweist, dadurch gekennzeichnet, daß innerhalb des Halters (1) und im wesentlichen parallel zur Mantelfläche des Kegelstumpfes eine Meßsonde (2) angeordnet ist, die als Sender und Empfänger für impulsförmige Signale ausgebildet ist und neben einem Impulsgeber auch eine zur Erfassung des Doppler-Effekts geeignete Auswerteeinrichtung aufweist.

2. Vorrichtung nach Anspruch 1, dadurch gekennzeichnet, daß im die gewölbte Auflagefläche (5) des Halters (1) umgebenden Rand (8) ein Drucksensor (9) angeordnet ist.

3. Vorrichtung nach einem der Ansprüche 1 oder 2, dadurch gekennzeichnet, daß der Halter (1) aus durchsichtigem Material besteht.

4. Vorrichtung nach Anspruch 3, dadurch gekennzeichnet, daß der Halter (1) aus Glas besteht.

5. Vorrichtung nach einem der Ansprüche 1 bis 3, dadurch gekennzeichnet, daß an der Auflagefläche (5) des Halters (1) zusätzlich ein Drucksensor (9) vorhanden ist.

## Claims

1. Device with an ultrasound or a laser measuring probe for checking of the eye and for the measurement and detection of functions and states thereof with use of a mount of substantially frustoconical shape placeable on the eye, wherein the cone tapers inwardly in direction towards the eye and has at the side of the eye a concave recess, which corresponds to the curvature of the eyeball, as support surface, characterised thereby that a measuring probe (2), which is constructed as transmitter and receiver for pulse-shaped signals and apart from a pulse transmitter also comprises an evaluating device suitable for detection of Doppler effect, is arranged within the mount (1) and substantially parallel to the circumferential surface of the cone frustum.

2. Device according to claim 1, characterised thereby that a pressure sensor (9) is arranged in the edge (8) surrounding the curved support surface (5) of the mount (1).

3. Device according to claim 1 or 2, characterised thereby that the mount (1) consists of transparent material.

4. Device according to claim 3, characterised thereby that the mount (1) consists of glass.

5. Device according to one of claims 1 to 3, characterised thereby that a pressure sensor (9) is additionally present at the support surface (5) of the mount (1).

## Revendications

1. Dispositif avec une sonde de mesure laser ou à ultrasons pour examiner les yeux et pour mesurer et saisir les fonctions et états de l'oeil en utilisant un support pouvant être posé sur l'oeil, sensiblement en forme de tronc de cône convergent en direction de l'oeil et présentant du côté de l'oeil un évidement concave qui correspond à la courbure du globe oculaire et qui sert de surface d'appui caractérisé par une sonde de mesure (2) disposée à l'intérieur du support (1) et sensiblement parallèle à l'enveloppe du cône, qui est conçue comme élément émetteur et récepteur de signaux sous forme d'impulsions et qui comprend, outre un émetteur d'impulsions, un dispositif d'évaluation approprié à la saisie de l'effet Doppler.

2. Dispositif selon la revendication 1 caractérisé par un capteur de pression (9) disposé dans le bord (8) entourant la surface d'appui courbe (5) du support (1).

3. Dispositif selon l'une des revendications 1 ou 2 caractérisé en ce que le support (1) est en matériau transparent.

4. Dispositif selon la revendication 3 caractérisé en ce que le support (1) est en verre.

5. Dispositif selon l'une des revendications 1 à 3 caractérisé en ce que il est prévu un capteur de pression (9) supplémentaire disposé sur la surface d'appui (5) du support (1).

FIG.1

EP 0 664 684 B1

FIG.2

6